Europäisches Patentamt

European Patent Office

Office européen des brevets

(19)

(11) **EP 0 554 381 B2**

(12) **NEW EUROPEAN PATENT SPECIFICATION**

(45) Date of publication and mention
of the opposition decision:
**26.04.2000 Bulletin 2000/17**

(45) Mention of the grant of the patent:
**17.01.1996 Bulletin 1996/03**

(21) Application number: **91920537.7**

(22) Date of filing: **21.10.1991**

(51) Int. Cl.[7]: **A61K 38/02**, A61K 38/20,
A61K 38/27

(86) International application number:
**PCT/US91/07759**

(87) International publication number:
**WO 92/07578 (14.05.1992 Gazette 1992/11)**

(54) **USE OF PROTECTIVE AGENTS AGAINST REACTIVE OXYGEN SPECIES**

VERWENDUNG GEGEN REAKTIVE SAUERSTOFFVERBINDUNGEN SCHÜTZENDER
WIRKSTOFFE

UTILISATION D'AGENTS PROTECTEURS CONTRE DES ESPECES D'OXYGENE REACTIVES

(84) Designated Contracting States:
**AT BE CH DE DK ES FR GB GR IT LI LU NL SE**

(30) Priority: **25.10.1990 US 602850**

(43) Date of publication of application:
**11.08.1993 Bulletin 1993/32**

(73) Proprietor: **GENENTECH, INC.**
**South San Francisco California 94080 (US)**

(72) Inventor: **WONG, Grace H. W.**
**South San Francisco, CA 94080 (US)**

(74) Representative:
**Nicholls, Kathryn Margaret et al**
**MEWBURN ELLIS**
**York House**
**23 Kingsway**
**London WC2B 6HP (GB)**

(56) References cited:
EP-A- 0 235 906       EP-A- 0 357 240
WO-A-91/14443       WO-A-91/15227
US-A- 4 861 587

• Dialog Information Services, file 155, Medline,
66-91, Dialog accession no. 06998107; Hellqvist
S et al.: "Interleukin i induces new protein
formation in isolated rat islets of Langerhans"
• Dialog Information Services, file 155, Medline 66-
91, Dialog accession no. 06866198; Zimmerman
RJ et al.: "Oxidative damage in murine tumor
cells treated in vitro by recombinant human
tumor necrosisfactor"

• Blood, 72(3), 1988, Neta, R. and Oppenheim, J.J.,
pp. 1093-1095
• J. Immunol., 136, 1986, Neta, R., Douches, S. and
Oppenheim, J.J., pp. 2483-2485
• J. Immunol., 140, 1988, Neta, R., Oppenheim, J.J.
and Douches, S.D., pp. 108-111
• Lymphokine Research, 6(3), 1987, Urbaschek,
R., Männel, D.N. and Urbaschek, B., pp. 179-186
• J. Appl. Physiol., 68(3), 1990, Tsan, M.-F., White,
J.E., Santana, T.A. and Ching, Y.L., pp. 1211-1219
• J. Clin. Invest., 79, 1987, White, C.W., Ghezzi, P.,
Dinarello, C.A., Caldwell, S.A., McMurtry, I.F. and
Repine, J.E., pp. 1868-1873
• A. Nowotny Ed., Plenum Press, 1983, Behling,
U.H., pp. 127-148
• Sience, 242, 1988, Wong, G.H.W. and Goeddel,
D.V., pp. 941-944
• Cell, 58, 1989, Wong, G.H.W., Elwell, J.H.,
Oberley, L.W. and Goeddel, D.V., pp. 923-931
• Lymphokine Research, 5 Supplement 1, 1986,
Neta, R., Vogel, S.N., Oppenheim, J.J. and
Douches, S.D., pp. S105-110
• Texas Rep. Biol. Med., 35, 1977, Lvovsky, E.,
Baze, W.B., Hilmas, D.E. and Levy, H.B., pp. 388-
393

EP 0 554 381 B2

## Description

BACKGROUND OF THE INVENTION

This invention relates to methods for inhibiting, preventing, protecting against or treating the deleterious effects of reactive oxygen species. The invention also relates to methods for protecting against injury to tissues from ischemia and reperfusion injury.

Superoxide radicals ($0_2$-) and other highly reactive oxygen species such as hydrogen peroxide ($H_20_2$) and hydroxyl radicals (referred to herein as reactive oxygen species or "ROS") are produced *in vivo* by enzymatic, spontaneous, and photochemical oxidation reactions. ROS are produced by mitochondria during electron transport. Other intracellular sources of $0_2^-$ and $H_20_2$ are endoplasmic reticulum, peroxisomes, and nuclear and plasma membranes. Examples of disorders associated with the generation of ROS include: synovial inflammation induced by bacterial lipopolysaccharide endotoxin (LPS), inflammation caused by adjuvant-induced arthritis, bleomycin-induced lung fibrosis, reperfusion injury, transplantation rejection, hyperoxia, and any diseases caused by oxygen and light. It has been suggested that ROS may be involved in hyperthermic cell injury (Omar *et al*., Cancer Res. 47:3473, 1987), and that thermosensitivity is linked to a low rate of free radical removal.

Certain agents are capable of inducing superoxide or other oxygen free radicals. Such ROS activity may be determined by commonly used methods, such as their ability to induce guinea pig alveolar macrophages to produce reactive oxygen metabolites which are measurable by spectrometer at $A_{550-540}$nm (Leurs *et al*., Biochemistry International 18 (2):295-299, 1989), or by known inflammatory or chemiluminescence test models. Agents which are known to enhance the production of ROS include but are not limited to the following commercially available compounds: inhibitors of glutathione synthesis such as buthionine sulfoximine, anthracyclines such as adriamycin (doxorubicin), adriamycinone (doxorubicinone), daunomycin, daunomycinone, daunorubicin, and daunorubicin derivatives such as 5-iminodaunorubicin, ubiquinone, Acid Blues 25,80, and 41, Acid Green 25, anthraquinone and its derivatives such as 2-bromoanthraquine, 1,2-dihydroxyanthraquinone, 1,8-diaminoanthraquinone, 2,6-diaminoanthraquinone, 1,5-dichloroanthraquinone, 1,2-diaminoanthraquinone, and 2-chloro-anthraquinone, quinizarin, anthrarufin, quilalizarin, aloe-emodin and related compounds such as 5-nitro-aloe-emodin, 5-amino-aloe-emodin, 2-allylaloe-emodin, averufin, kalafungin, alizarin complexone dihydrate, quercetin dihydrate, acid black 48, procytoxid, leucotrofina, azimexon, and methoxycin-namonitrile. These ROS inducing agents may be administered therapeutically, by intravenous or other methods as desired.

A group of metalloproteins known as superoxide dismutases (SOD) catalyze the oxidation-reduction reaction

$$2O_2^- + 2 H^+ \rightarrow H_2O_2 + O_2$$

and thus provide part of the defense mechanism against oxygen toxicity. Eukaryotic cells contain copper-zinc SOD, which is found predominantly in the cytosol, and MnSOD, which is found mainly in mitochondria. Extracellular SOD is found primarily in extracellular fluids such as plasma, lymph, and synovial fluid, but occurs also in tissues (Hjalmarsson *et al*., Proc. Natl. Acad. Sci. USA 84:6340, 1987).

The scientific literature suggests that SOD administration may be useful in a wide range of clinical applications. Potential applications include prevention of oncogenesis and of tumor promotion, treatment of inflammations, reduction of the cytotoxic and cardiotoxic effects of anticancer drugs, protection of ischemic tissues and protection of spermatozoa (EPO Appl. EP 0 284 105 A2). It has also been suggested that oxygen free radicals are involved in the pathogenesis of, and that SOD administration protects against, influenza virus infection (Oda *et al*., Science 244: 974-976, 1989).

Ischemia causes injury to cells, and if continued for a sufficient length of time, can kill them. Reperfusion after a brief period of ischemia, although beneficial in the long term, frequently results in an initial injury to the tissues upon reoxygenation, presumably through the formation and involvement of reactive oxygen species. This phenomenon has been described in the literature with heart, skin, intestine, pancreas, and variety of tissues. It is also important to protect against reoxygenation injury during thrombolytic therapy, and in the preservation of organs for transplantation.

Growth hormone (GH) and human growth hormone (hGH) are secreted in the pituitary. The mature form of hGH consists of 191 amino acids and has a molecular weight of about 22,000; its sequence and characteristics are set forth, for example, in Hormone Drugs, Geuriguian *et al*., U.S.P. Convention, Rockville Md (1982).

hGH has been used for the treatment of hypopituitary dwarfism, and has been proposed for the treatment of burns, wound healing, dystrophy, bone knitting, diffuse gastric bleeding and pseudarthrosis. The major biological effect of GH is to promote growth. The organ systems affected include the skeleton, connective tissue, muscles, and viscera such as liver, intestine, and kidneys. Growth hormone exerts its action through interaction with specific receptors on cell membranes. Administration of growth hormone for treatment of pulmonary dysfunction and ventilator dependency has been proposed in PCT WO90/09189 published 23 August 1990; in this application causes of pulmonary dysfunction are stated to include physical injury as well as naturally occurring diseases such as pulmonary obstruction, pneumonia, asthma, emphysema, cancers, autoimmune diseases, neurological disorders including stroke, and chest deformities.

Interleukin-1 (IL-1) is produced by activated macrophages. At least two types exist, designated $\alpha$ and $\beta$ (March *et al*., Nature 315:641-647 (1985)). IL-1 mediates a wide range of biological activities; it has been found to stimulate fibroblast proliferation and to induce in these cells the synthesis of collagenase, prostaglandin $E_2$ and interferon beta-2, to decrease in adipocytes the activity of lipoprotein lipase, and to activate osteoclasts.

Tumor necrosis factors (TNFs) are polypeptides produced by mitogen-stimulated macrophages (TNF-$\alpha$) or lymphocytes (TNF-$\beta$) which are cytotoxic to certain malignantly transformed cells but not to certain normal cells (E. A. Carswell *et al*., Proc. Natl. Acad. Sci. U.S.A. 72:3666, 1975; B. J. Sugarman *et al*., Science 230:943, 1985; Schultze *et al*., J. Immunol. 140: 3000, 1988). TNF-$\alpha$ has been suggested to be responsible for wasting and cachexia in patients with cancer or severe infections, and both TNF-$\alpha$ and TNF-$\beta$ mediate many other biological effects. TNF is also known to induce MHC antigens. The inventors herein have reported that TNF induces MnSOD in various transformed and normal cell lines (Wong *et al*., Science 242:941, 1988).

TNF at certain dosages is known to trigger the generation of ROS in macrophages or neutrophils (Tsujimoto *et al*., Biochem. Biophys. Res. Commun. 137:1094, 1986; Matsubara *et al*., J. Immunol. 137:3295, 1986; Shalaby *et al*., Leuk. Biol. 41:196,1987; Berkow *et al*., J. Immunol. 139:3783, 1987). It has been suggested repeatedly in the literature that TNF at certain dosages enhances tissue injury caused by reactive oxygen species (see, e.g. Clark *et al*., J. Cell Biochem. Suppl. 12A, p. 40, Jan 1988; Sullivan *et al*., Infect. and Immunity 56(7): 1722-1729, 1988; and Tiegs *et al*., Biochem. Pharmacol. 38(4): 627-631, 1989).

The literature has reported that TNF-$\alpha$ and other cytokines such as IL-1 may protect against the deleterious effects of ionizing radiation produced during a course of radiotherapy, such as denaturation of enzymes, lipid peroxidation, and DNA damage (Neta *et al*., J. Immunol. 136(7):2483, 1987; Neta *et al*., Lymphokine Res. 5 *et al*., *supra*; Neta *et al*., Fed. Proc. 46:1200 (abstract), 1987; Urbaschek *et al*., Lymphokine Res. 6:179, 1987; U.S. Patent No. 4,861,587; Neta *et al*., J. Immunol. 140:108, 1988), and that TNF treatment accelerates restoration of hematopoiesis in animals compromised by sublethal doses of cytotoxic drugs or irradiation (Neta, *et al*., Blood 72(3):1093, 1988). A recent article reported that pretreatment with TNF protects mice from lethal bacterial infection (Cross *et al*., J. Exp. Med. 169:2021-2027, 1989). It has also been suggested that administration of subdeleterious amounts of TNF and/or IL-1 may modulate the deleterious effect of subsequent TNF and/or IL-1 administration; this reference further suggests that ionizing radiation may be administered as a sensitizing agent (EPO Appl. EP 0 259 863 A2). It has also been reported that pretreatment of cells with low levels of either TNF or IL-1 can confer resistance to killing by subsequent treatment with TNF-$\alpha$ and cycloheximide in combination (Wallach, J. Immunol. 132:2464, 1984; Hahn *et al*., Proc. Natl. Acad. Sci. U.S.A. 82:3814, 1985; Holtamann *et al*., J. Immunol 139:1161, 1987). It has been suggested that inadequate endogenous levels of TNF may be involved in the development of diabetes and of lupus erythematosus (Jacob *et al*., Nature 331:356-358, 1988).

Saez *et al*., Proc. Natl. Acad. Sci. USA 84:3056-3059, 1987, have shown that SOD protects cultured neurons against death by glucose deprivation. Tsan *et al*., J. Appl. Physiol. 68(3): 1211-1219, 1990, has recently confirmed that tracheal insufflation of TNF (and IL-1 in unpublished data) protects rats against oxygen toxicity.

White *et al*., J. Clin. Invest, 79:1868-1873, 1987, have shown that pretreatment with a combination of TNF and IL-1 decreases lung injury and mortality in rats exposed to hyperoxia, but indicate that, with i.p. injection, either TNF or IL-1 alone protected the rats only comparable to saline.

It is an object of this invention to provide methods for protecting ischemic tissues, such as those tissues, bones or organs to be transplanted from a donor to a recipient patient, or those tissues whose oxygen supply has been blocked, from the effects of ROS.

It is a further object herein to provide methods to inhibit, prevent or treat reperfusion injury, bronchopulmonary dysplasia, stroke, arteriolosclerosis, atherosclerosis, myocardial infarct, inflammatory autoimmune diseases, viral infection, inflammation-induced arthritis, hyperoxia, sepsis, diabetes, influenza, multiple sclerosis, hyperbaric treatment after premature birth, acquired immunodeficiency syndrome, transplant rejection or transplantation injury, bleomycin-induced lung fibrosis, synovial inflammation induced by bacterial LPS endotoxin, lung injury resulting from immune complexes, hyperbaric treatment of cancer, kidney disease, Parkinson's disease, sickle cell anemia, sickle cell trait, alcoholic or non-alcoholic cirrhosis, or other diseases associated with toxic ROS.

Another object of this invention is to supply perfusion solutions and excised, perfused tissues for transplant.

These and other objects of the invention will be apparent from consideration of the specification as a whole.

## SUMMARY OF INVENTION

According to the present invention there is provided the use of a protective agent in the production of a composition for the prophylaxis and/or treatment of the deleterious effects of reactive oxygen species in a patient, said protective agent being selected from:

> (a) growth hormone ('GH')
> (b) tumor necrosis factor ('TNF') and GH
> (c) interleukin-1 ("IL-1") and GH
> (d) IL-1 and TNF and GH.

Unexpectedly, and contrary to the suggestions in the literature, the present inventors have discovered that treatment with certain doses of IL-1 and/or TNF in combination with growth hormone protect against ROS damage.

The objects of this invention are accomplished by methods utilizing these effects of the protective agent(s) TNF, IL-1 and GH. In one preferred method, a patient at risk for reperfusion injury is treated with an effective but sub-deleterious amount of at least one of these agents before, at, or after, but preferably prior to reperfusion. In another embodiment, a patient with a condition or disease associated with ROS (as described above) is treated with a dose of at least one of these agents which is effective at preventing, inhibiting or treating that condition.

Other preferred embodiments relate to tissue transplant. In one embodiment, ischemic tissue is protected by the administration of an effective amount of at least one of (a) GH, (b) DF, (c) TNF, or (d) IL-1 and/or TNF, plus GH and/or DF prior to a reperfusion of the tissue. This ischemic tissue may be treated prior to or after removal from a donor, and before or after it is transferred to or implanted in a recipient patient.

This invention also encompasses perfusion solutions comprising at least one of the above protective agent(s) in a pharmaceutically acceptable excipient, as well as perfused, excised tissues, where the tissues are perfused with such a perfusion solution.

In an alternate embodiment, the protective agent(s) is administered to a tissue donor prior to preparation for removal of the tissue.

## DESCRIPTION OF THE DRAWINGS

FIGURE 1 shows that TNF pretreatment protects against heart damage due to ischemia and reperfusion.

FIGURE 2 shows the induction of MnSOD in ischemic rat hearts pretreated with TNF prior to reperfusion.

FIGURE 3A illustrates the results obtained in Example 2, showing that TNF-$\alpha$ and DF protect adult rats from oxygen toxicity.

FIGURE 3B shows the synergistic effect of DF plus TNF.

FIGURE 4 illustrates the results obtained in Example 2, showing that TNF-$\alpha$ and GH protect adult rats from oxygen toxicity.

FIGURE 5 shows the protective effect of TNF on neuronal survival after glucose deprivation. The number of neurons dying under each condition (10,000 total neurons per dish) are shown.

## DETAILED DESCRIPTION

Tumor necrosis factor or TNF, as employed herein, refers in general to the various forms of TNF which exhibit one or more biologic properties of tumor necrosis such as tumor cell lysis, inhibition of infectious agents, MHC antigen induction, and neutralization by antibody to TNF-$\alpha$ or TNF-$\beta$ but not by antibodies to other cytokines. It is believed that gamma interferon is synergistic with TNF in anti-tumor or anti-viral assays for TNF, and may therefore be desirably administered along with TNF in the practice of this invention.

In particular, the tumor necrosis factors useful herein include TNF-$\alpha$ and TNF-$\beta$. The former is described in copending EPO Appl. EP 0 168 214 A2 together with methods for its synthesis in recombinant cell culture. Similarly, the latter (previously called lymphotoxin) and suitable recombinant synthesis methods are described in copending EPO Appl. EP 0 164 965 A2. The TNF-$\alpha$ and TNF-$\beta$ described in these applications include cytotoxic amino acid sequence and glycosylation variants which also are used herein. Of course, TNF-$\alpha$ or TNF-$\beta$ from non-recombinant sources are also useful in the practice of this invention.

TNF-$\alpha$ or TNF-$\beta$ are used alone or in admixture with one another in proportions empirically determined to exert the most effective clinical response. TNF is not species specific, so TNFs from other animal species, e.g. porcine or bovine, are useful for treatment of humans. The preferred TNF for treatment of humans is mature human TNF-$\alpha$ from recombinant microbial cell culture. The TNF ordinarily will have a cytolytic activity on susceptible L-929 murine cells of greater than about $1 \times 10^6$ units/mg, wherein a unit is defined as set forth in the above-described patent applications.

As used herein, the terms "growth hormone" or "GH" denote growth hormone produced from natural source extraction and purification, as well as by recombinant cell culture systems. "hGH" refers to human growth hormone.

See, for example, U.S. Patent 4,321,832. The terms likewise cover biologically active human growth hormone equivalents, e.g., those differing by one or more amino acids(s) in the overall sequence. Further, the terms as used in this application are intended to cover substitution, deletion and insertion amino acid variants of GH, or post-translational modifications.

As used herein, "IL-1" denotes interleukin-1 produced from natural source extraction and purification, as well as by recombinant cell culture systems. See, for example, March *et al*., Nature 315:641-646 (1985)). The terms likewise cover biologically active IL-1 equivalents, e.g., those differing by one or more amino acids(s) in the overall sequence. Further, the terms as used in this application are intended to cover substitution, deletion and insertion amino acid variants of IL-1, or post-translational modifications.

The mature amino acid sequences for human (H) and murine (M) DF are disclosed in EP 285,448, published 5 Oct. 1988, especially at Fig. 26, including methods for its production in recombinant cell culture. See also D. P. Gearing *et al*., Nucleic Acids Res. 16:9857(1988), and N. M. Gough *et al*., Proc. Nat. Acad. Sci. USA 85:2623-2627 (1988).

The cytoprotective agents discussed herein, GH, TNF and IL-1, include glycosylation variants as well as unglycosylated forms of the agents, fusions of the agents with heterologous polypeptides, and fragments of the agents, so long as the variants possess the requisite cytoprotective activity.

The formulations may contain compounds previously suggested for use in the treatment of the conditions and diseases described herein, as well as those compounds previously suggested for use in preventing damage from ROS. Antioxidants, such as ascorbate, fibrinolytic agents such as tissue plasminogen activator, and other compounds previously suggested for use in preventing reperfusion injury may also be included. Agents that block the toxicity of high doses of TNF and/or IL-1--such as glucocorticoids and indomethacin--without altering the MnSOD-inducing activity of TNF--are also utilized in the practice of this invention. Agents which block induction of MHC antigens are also desirably administered. Compounds which effect the redox potential of ROS may also be utilized in a formulation. The protective agent(s) also is suitably formulated together with known agents in order to modify or enhance half-life or therapeutic activity. These other agents or therapies are used at the same time as the GH and optionally TNF and/or IL-1 is administered or in a sequential course of therapy.

The TNF, GH, and/or IL-1 are placed into sterile, isotonic formulations together with required cofactors. Formulations may contain one or more protective agents. The formulations are preferably liquid, and ordinarily a physiologic salt solution or dextrose solution, together with conventional stabilizers and/or incipients. Compositions may also be provided as lyophilized powder for ultimate delivery in solution. Saline is a suitable carrier, although other conventional parenteral solutions or buffers are usable.

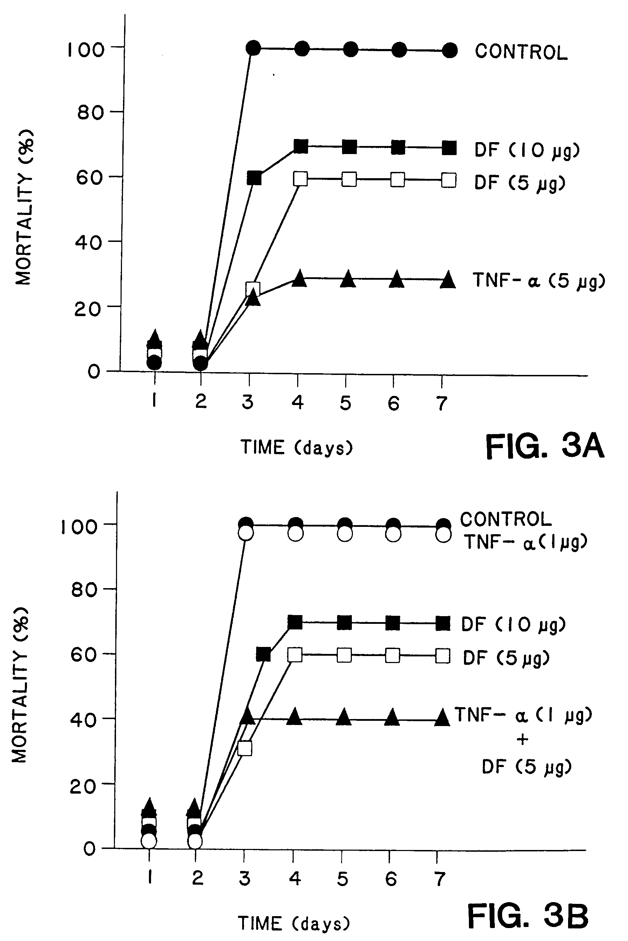

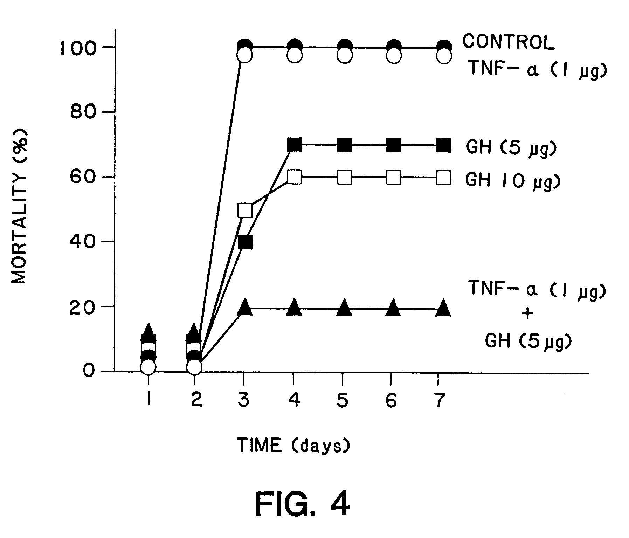

In a pharmacologic sense, in the context of the present invention, a therapeutically effective amount of TNF, GH, and/or IL-1 refers to that amount effective to protect normal cells from the deleterious effects of ROS. It has been discovered that TNF and/or IL-1 plus GH, provide synergistic protection against oxygen free-radical damage such as caused by hyperoxia, and thus relatively smaller dosages of a particular agent may be administered (See Figs. 3 and 4).

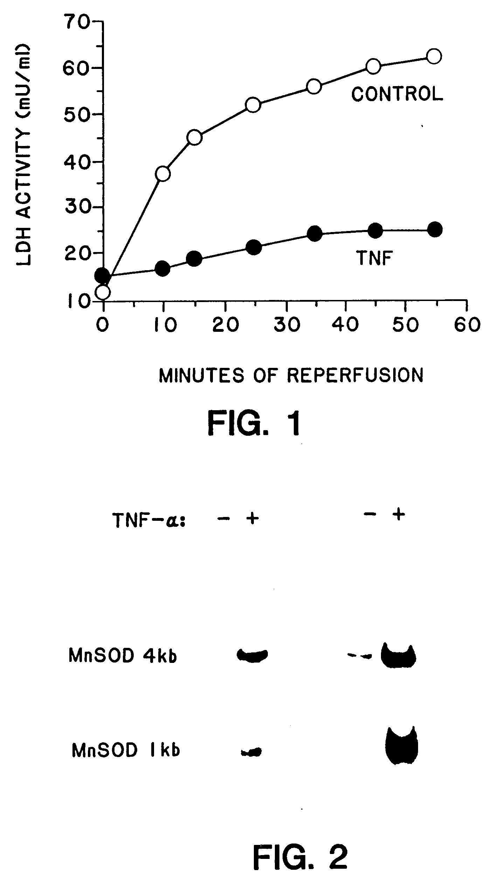

Although it is currently believed that induction of MnSOD is not sufficient to protect cells from damage, monitoring of its induction by TNF is a convenient indication of TNF activity under this invention. As shown in Fig. 2, TNF induces MnSOD in ischemic rat hearts pretreated with TNF before reperfusion.

The therapeutically effective dosage of TNF to be administered to a human patient or human tissue generally will range from about 1-250 $\mu g/m^2$ per dose, and preferably from about 1-10 $\mu g/m^2$, and most preferably 10$\mu g/m^2$, although the dose of the TNF administered will be dependent upon the species of the patient, the properties of the TNF employed, e.g. its activity and biological half-life, the concentration of the TNF in the formulation, the rate of dosage, the clinical tolerance of the patients involved, the pathological condition of the patients and the like, as is well within the skill of the physician. It will be appreciated that the practitioner will adjust the therapeutic dose in line with clinical experience for any given TNF. Preferably, the TNF is administered intravenously or intramuscularly.

The amount of GH, TNF and/or IL-1 which is used will depend upon the therapeutic protocol, considering the use of hyperoxia, irradiation or chemotherapy, the condition of the patient, the activity of the agent used, the administration route, and other influences that will be appreciated by the ordinary artisan. Treatment effective amounts of IL-1 administered to mice range between about 0.5 to 25 nanograms per gram of body weight of mouse.

For hGH, a suitable dosage for human administration ranges from 0.001 mg per kg of bodyweight per day to about 0.2 mg per kg of bodyweight per day. Generally, daily dosages of GH will be from about 0.05 mg per kg of bodyweight per day. Normally, from 0.07 to 0.15 mg/kg, in one or more applications per day, is effective to obtain the desired result. In an alternative approach, the GH, particularly where formulated in a timed-release form, may be administered less frequently, i.e., every other day or every third day for certain indications. It is presently preferred the GH be administered within 0 to 24 hours following exposure to free-radical injury.

In the practice of this invention, compositions which include a therapeutically effective amount of GH and optionally TNF, and/or IL-1 are administered to patients having or at risk for damage from ROS. Accordingly, in some embodiments of this invention, the protective agent, alone or with other agents as described above, is administered to a patient within a temporal period, most preferably within 24 hours prior, preferably prior to or concurrent with, or shortly following exposure, to ROS. In the case of reperfusion, exposure to the ROS may be predetermined and deliberate, or may be a consequence of other therapeutic measures.

In other embodiments, a patient's ROS exposure is of a more chronic or regular nature, as with certain of the diseases and chronic conditions described above. In these situations, the protective agent(s) may be administered as part of a long-term course of therapy.

Some embodiments deal with the transfer to and implantation of tissues, and the complex tissues known as organs, in a recipient patient. For purposes of this invention, the term "tissues" shall be understood to include, without limitation, muscle tissue, connective tissue, epithelial tissue, nervous tissue, vascular tissue, bone, brain, reproductive organs, respiratory organs, digestive organs, excretory organs, urinary organs, sensory organs, and skeletal muscle organs. Particularly preferred tissues include heart, lung, kidney, liver, skin and bone grafts. Suitable tissues are synthetic as well as those which are removed from a donor.

For tissues to be removed from a donor prior to their transfer to and implantation in a patient, treatment with GH and optionally TNF and/or IL-1 may be a accomplished in several different ways. The protective agent(s) may be administered to the donor, as described above. Alternatively, the tissue itself may be treated, either prior to or after removal from the donor, and either prior to or after transfer to the patient, but most preferably prior to or concurrent with reperfusion.

Since the first successful human orthotopic liver transplant by Starzl in 1967, methods for the transplant of tissues, as well as suitable protocols for their perfusion with various agents, have become commonly known in the art. Suitable protocols and perfusion solutions are described, for example, in Kalayoglu *et al*., The Lancet, March 19, 1988, pp. 617-619. Perfusion is commonly accomplished with a mechanical pump, as described in Example 1 below. Suitable perfusion solutions include lactated Ringer's solution, UW solution, and pharmaceutically acceptable isotonic solutions. These solutions enable a TNF, GH, and/or IL-1 -treated tissue to be preserved by continuous perfusion or cold storage until it is implanted into a recipient patient.

In an embodiment of this invention, perfusion solutions are provided, comprising GH and optionally TNF and/or IL-1 at concentrations of approximately 1 - 250 $\mu g/m^2$ with a pharmaceutically acceptable isotonic solution.

For each type of type of disease or injury, and for each animal species to be treated, the exact protective TNF, GH, and/or IL-1 dosage necessary for this protective effect may vary from that shown herein, and the exact administration parameters suitable for any given patient or animal species will be determined by routine experimentation. Typically, the patient or tissue is first administered the agent, e.g. by intravenous or intramuscular administration, and thereafter exposed to ROS. The patient or tissue is monitored for symptoms of any beneficial or deleterious effects of the treatment. If the initial treatment is partially successful or unsuccessful, the process may be repeated, optionally with a modification of the dosage or route of administration.

According to this invention, patients from differing species are all treated by the pharmaceutically acceptable administration of TNF, GH, and/or IL-1 in a pharmaceutically effective dosage and for a period of time sufficient to inhibit, prevent, protect from the damaging effects of ROS.

It is also envisioned that, in the practice of this invention, administration of the protective agent(s) may by accompanied by the therapeutic administration of a course of radiation, heat, and/or ROS inducing agents. The agent, alone or with other agents, may be administered to a patient prior to, following, or simultaneously with exposure to radiation, heat, or ROS inducing agents. Exposure to the radiation, heat, or ROS inducing agents may be predetermined and deliberate, or may be a consequence of other therapeutic measures. Heat or ROS-inducing agents may be administered with radiation, and/or with an additional dose of TNF, GH and/or IL-1.

ROS inducing agents and their methods of administration are described above. Radiation and heat are administered by protocols commonly known to practitioners, as described in the literature cited above. Typically, radiation is given in pulses over a period of time from 1 - 8 weeks, for a total dose of approximately 1000-1200 rads. Heat may be administered by known methods such as a heat blanket or hyperthermia chamber, for a period sufficient to raise a patient's body tissue temperature above 37 °C, preferably 40 - 45 °C, and most preferably to approximately 42 °C.

Where GH alone is administered to a patient having or at risk of hyperoxia damage, the patient may be subjected to atmospheric conditions with greater levels of oxygen than normal atmospheric conditions.

In order that the invention described herein may be more fully understood, the following examples are set forth. It should be understood that these examples are for illustrative purposes only, and are not to be construed as limiting this invention in any manner.

EXAMPLE 1

The effect of TNF on isolated rat heart.

This experiment was conducted by Dr. Lynn Eddy at the University of Southern California. Healthy Sprague Dawley rats weighing between 200 and 300g are anesthetized with sodium pentobarbital, 30 mg/kg, intraperitoneally.

Heparin (500 U) is administered intravenously. The chests are opened and the hearts rapidly excised and placed in iced cold buffer until they stop beating. The hearts are mounted by the aortic roots to a stainless steel cannula and perfused through the aorta in a retrograde manner (Langendorff). Krebs-Henseleit buffer of the following composition is used throughout the experiments (final concentration in mM/L): NaCl, 118; KCl, 4.7; CaCl, 2.5; MgSO, 1.2; KH PO, 1.2; CaEDTA, 0.5; NaHCO, 25; and glucose, 10. The buffer is aerated with 95% 0 - 5% CO. Perfusion is maintained by a peristaltic pump at a flow rate of 10 ml/min and perfusion pressure is monitored by a P23AA Statham pressure transducer attached to a side arm of the aortic inflow cannula and recorded on a Hewlett Packard 7702B recorder. The complete perfusion system is maintained within a thermostatically controlled plexiglass chamber maintained at 37°C.

The hearts are perfused 3-4 minutes to remove blood. After a 20 minute control period, global ischemia is initiated by stopping the pump and turning off the 95% 0 - 5% CO. At the end of the ischemic period, the pump is restarted and the buffer reoxygenated.

Enzyme leakage from the heart cells is determined in effluent samples collected from the heart at specific time periods during the control period and during reperfusion. Lactic dehydrogenase (LDH) activity is assayed by monitoring the oxidation of NADH, using pyruvate as the substrate. NADH is monitored at 340 nm using a Perkin Elmer Lambda 3 recording spectrophotometer. As shown in Fig. 1, LDH activity from hearts treated with 10μg TNF compared to controls indicates that the TNF protected the heart from damage mediated by its ischemia and reperfusion.

EXAMPLE 2

This experiment was conducted by Dr. Min-Fu Tsan, at the Department of Veterans Affairs Medical Center in Albany, New York. The effects of TNF-alpha, TNF-beta, IL-1 beta, interferon (IFN)-gamma, growth hormone and DF, alone and in various combinations, on the protection of rats against oxygen toxicity was examined.

Tracheal insufflation of the cytoprotective agent and the exposure of the rats to hyperoxia was performed essentially as described in Tsan *et al*., J. Appl. Physiol. 68:1211-1219, 1990. Briefly, male Sprague-Dawley rats (Harlan Sprague Dawley, Altamont, NY, USA) free of respiratory infections and weighing between 250-350 g, were anesthetized with methoxyflurane (Pitman-Moore Inc., Washington Crossing, NJ, USA) and were intubated with a 16 G i.v. catheter (Angiocath, Becton Dickinson, Sandy, Utah, USA). One ml of the protective agent(s) (agents and concentrations are shown in the next paragraph) was administered to the rats in calcium-magnesium free Hanks' balanced salt solution (HBSS) (or 1 ml HBSS for control animals) followed by 2 ml of air, injected through the intratracheal catheter. Auscultation of the chest with a stethoscope was done to insure that the agent was insufflated into the lungs. After the rats had recovered from the effect of anesthesia, they were placed in groups of 5 in a lucite chamber (45 by 40 by 25 cm$^3$) which was flushed with 100 % $O_2$ at 15 liter/min for 5 min and then maintained at 5 liter/min. The concentration of $O_2$ in the chamber as monitored using an oxygen analyzer (Hudson Oxygen Analyzer, Ventronics Products Division, Temecula, CA, USA) was more than 95 % at all times. The rats were given free access to water and diet. Control exposure (normoxia) was performed in room air.

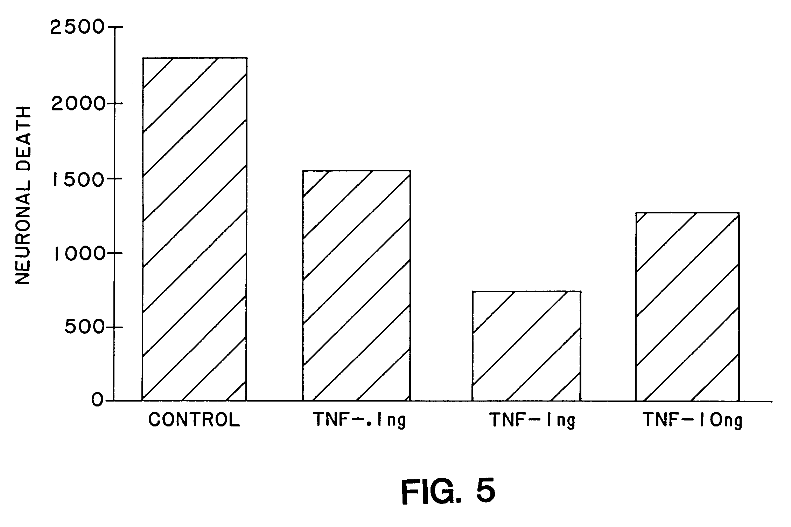

Results are shown in Figures 4 and 5, and the percent survival after 3 days and 7 days are shown below.

| Tracheal insufflate | n | 3 days | 7 days |
|---|---|---|---|
| HBSS (control) | 45 | 0 | 0 |
| DF 5 μg | 10 | 70 | 40 |
| DF 10 μg | 10 | 40 | 30 |
| GH 5 μg | 15 | 53 | 27 |
| GH 10 μg | 10 | 50 | 40 |
| TNF-α 5 μg | 17 | 76 | 71 |
| TNF-α 1 μg | 10 | 0 | 0 |
| DF 5 μg + TNF 1 μg | 15 | 100 | 93 |
| GH 5 μg + TNF 1 μg | 15 | 67 | 67 |
| | * | * * | * |

In data not shown, IL-1 was also protective comparable to TNF. It may be seen from these results that TNF or IL-1 alone are effective in preventing death due to hyperoxia. Growth Hormone and DF are less effective for the same purpose when used alone, but synergize with TNF and/or IL-1 in protecting the animals. This work indicates that these agents be used in any hyperbaric treatment, including oxygen treatment of premature infants and cancer patients.

Example 3

This experiment was conducted by Dr. John A. Kessler, at the Albert Einstein College of Medicine of Yeshiva University, New York New York. The protective effect of TNF on neuronal survival after glucose deprivation (analogous to nutrient depletion following ischemia) was studied according to the method of Saez *et al*., Proc. Natl. Acad. Sci. USA 84: 3056-3059, 1987. As shown in Fig.5, TNF was found to give a strongly protective effect; maximal effects are shown at 1 ng/ml of TNF.

**Claims**

1. The use of a protective agent in the production of a composition for the prophylaxis and/or treatment of the deleterious effects of reactive oxygen species in a patient, said protective agent being selected from:

    (a) growth hormone ('GH')
    (b) tumor necrosis factor ('TNF') and GH
    (c) interleukin-1 ("IL-1") and GH
    (d) IL-1 and TNF and GH.

2. The use according to claim 1, wherein said patient has or is at risk of having reperfusion injury, bronchopulmonary dysplasia, stroke, arteriolosclerosis, atherosclerosis, myocardial infarct, sepsis, acquired immunodeficiency syndrome, diabetes, multiple sclerosis, inflammation-induced arthritis, hyperbaric treatment of premature birth, hyperoxia, transplant rejection or transplantation injury, sickle cell anaemia, sickle cell trait, hyperbaric cancer treatment, alcoholic or non-alcoholic cirrhosis, bleomycin-induced lung fibrosis, synovial inflammation induced by bacterial LPS endotoxin, lung injury resulting from immune complexes, or Parkinson's disease.

3. The use according to claim 1 or claim 2, wherein said composition also comprises alpha, beta, or gamma-interferon.

4. The use according to claim 1 or claim 2, wherein said tumor necrosis factor is tumor necrosis factor-$\alpha$ or tumor necrosis factor-$\beta$.

5. The use according to claim 1, wherein said composition also comprises a fibrinolytic agent or antioxidant.

6. The use of a protective agent(s) selected from those set out in claim 1, in the production of a composition suitable for the protection of ischemic tissues.

7. The use according to claim 6, wherein said composition also comprises alpha, beta, or gamma-interferon.

8. The use according to claim 6, wherein said tumor necrosis factor is tumor necrosis factor-$\alpha$ or tumor necrosis factor-$\beta$.

9. The use according to claim 6, wherein said composition is formulated for perfusion into said tissue.

10. The use according to claim 6, wherein said composition also comprises a pharmaceutically acceptable excipient.

11. The use according to claim 6, wherein said composition further comprises an antioxidant or fibrinolytic agent.

12. The use according to claim 6, wherein said tissue is selected from the group consisting of muscle tissue, connective tissue, epithelial tissue, nervous tissue, vascular tissue, bone, brain, reproductive organs, respiratory organs, digestive organs, excretory organs, urinary organs, sensory organs, and skeletal muscle organs.

13. A perfused, excised tissue intended for transplant comprising a tissue and a perfusion solution, said solution comprising a protective agent selected from those set out in claim 1.

14. The tissue according to claim 13 wherein said tumor necrosis factor (TNF) is TNF-$\alpha$ or TNF-$\beta$.

**Patentansprüche**

1. Verwendung eines oder mehrerer Schutzmittel zur Herstellung einer Zusammensetzung zur Prophylaxe gegen die und/oder Behandlung der schädlichen Wirkungen von reaktiven Formen des Sauerstoffs bei einem Patienten, wobei das Schutzmittel ausgewählt ist aus:

   (a) Wachstumshormon (GH),
   (b) Tumornekrosefaktor (TNF) und GH,
   (c) Interleukin-1 (IL-1) und GH,
   (d) IL-1 und TNF und GH.

2. Verwendung nach Anspruch 1, worin der Patient an Reperfusionsverletzungen, bronchopulmonaler Dysplasie, Schlaganfall, Arteriosklerose, Atherosklerose, Myocardinfarkt, Blutvergiftung, AIDS, Diabetes, multipler Sklerose, entzündungsbedingter Arthritis, hyperbarischer Behandlung bei Frühgeburten, Hyperoxie, Transplantatabstoßung oder Transplantationsverletzung, Sichelzellenanämie, Sichelzellenmerkmal, hyperbarischer Krebsbehandlung, alkohol- oder nicht-alkoholbedingter Zirrhose, bleomycin-induzierter Lungenfibrose, durch bakterielles LPS-Endotoxin herbeigeführter Synovialentzündung, von Immunkomplexen herrührender Lungenverletzung oder Parkinson-Syndrom leidet oder davon bedroht ist.

3. Verwendung nach Anspruch 1 oder 2, worin die Zusammensetzung auch α-, β-oder γ-Interferon umfaßt.

4. Verwendung nach Anspruch 1 oder 2, worin der Tumornekrosefaktor Tumornekrosefaktor-α oder Tumornekrosefaktor-β ist.

5. Verwendung nach Anspruch 1, worin die Zusammensetzung auch ein fibrinolytisches Mittel oder ein Antioxidans umfaßt.

6. Verwendung eines oder mehrerer Schutzmittel, ausgewählt-aus den in Anspruch 1 angeführten, zur Herstellung einer Zusammensetzung, die sich zum Schutz von ischämischen Geweben eignet.

7. Verwendung nach Anspruch 6, worin die Zusammensetzung auch α-, β- oder γ-Interferon umfaßt.

8. Verwendung nach Anspruch 6, worin der Tumornekrosefaktor Tumornekrosefaktor-α oder Tumornekrosefaktor-β ist.

9. Verwendung nach Anspruch 6, worin die Zusammensetzung zur Perfusion in das Gewebe formuliert ist.

10. Verwendung nach Anspruch 6, worin die Zusammensetzung auch einen pharmazeutisch annehmbaren Exzipienten umfaßt.

11. Verwendung nach Anspruch 6, worin die Zusammensetzung auch ein Antioxidans oder ein fibrinolytisches Mittel umfaßt.

12. Verwendung nach Anspruch 6, worin das Gewebe aus der Gruppe ausgewählt ist, die aus Muskelgewebe, Bindegewebe, Epithelgewebe, Nervengewebe, Gefäßgewebe, Knochen, Gehirn, Geschlechtsorganen, Atmungsorganen, Verdauungsorganen, Ausscheidungsorganen, dein Harnapparat, Sinnesorganen und Skelettmuskelorganen besteht.

13. Perfundiertes, exzidiertes Gewebe, das für ein ein Gewebe und eine Perfusionslösung umfassendes Transplantat bestimmt ist, wobei die Lösung ein oder mehrere Schutzmittel, ausgewählt aus den in Anspruch 1 angeführten, umfaßt.

14. Gewebe nach Anspruch 13, worin der Tumornekrosefaktor (TNF) TNF-α oder TNF-β ist.

**Revendications**

1. Utilisation d'un agent protecteur dans la production d'une composition pour la prophylaxie et/ou le traitement des effets néfastes d'entités réactives dérivées de l'oxygène chez un patient, cet agent protecteur étant choisi entre :

(a) l'hormone de croissance ('GH')

(b) le facteur de nécrose de tumeur ('TNF') et la GH

(c) l'interleukine-1- ("IL-1") et la GH

(d) la IL-1 et le TNF et la GH.

2. Utilisation suivant la revendication 1, dans laquelle le patient souffre, ou présente un risque, de lésions de reperfusion, de dysplasie brochopulmonaire, d'ictus, d'artériosclérose, d'athérosclérose, d'infarctus du myocarde, de septicémie, de syndrome d'immunodéficience acquise, de diabète, de sclérose en plaques, d'arthrite induite par l'inflammation, des conséquences du traitement hyperbare d'un prématuré, d'hyperoxie, d'un rejet de greffe ou d'une lésion de transplantation, d'anémie à hématies falciformes, d'un trait thalassémique, des conséquences du traitement hyperbare d'un cancer, des conséquences du traitement de la cirrhose alcoolique ou non alcoolique, de fibrose pulmonaire induite par la bléomycine, d'inflammation synoviale induite par l'endotoxine LPS bactérienne, d'une lésion pulmonaire résultant de complexes immuns ou de maladie de Parkinson.

3. Utilisation suivant la revendication 1 ou la revendication 2, dans laquelle la composition comprend également de l'interféron alpha, bêta ou gamma.

4. Utilisation suivant la revendication 1 ou la revendication 2, dans laquelle le facteur de nécrose tumorale est le facteur de nécrose de tumeur $\alpha$ ou le facteur de nécrose de tumeur $\beta$.

5. Utilisation suivant la revendication 1, dans laquelle la composition comprend également un agent fibrilotytique ou un anti-oxydant.

6. Utilisation d'un ou plusieurs agents protecteurs choisis entre ceux indiqués dans la revendication 1, dans la production d'une composition apte à la protection de tissus ischémiques.

7. Utilisation suivant la revendication 6, dans laquelle la composition comprend également de l'interféron alpha, bêta ou gamma.

8. Utilisation suivant la revendication 6, dans laquelle le facteur de nécrose de tumeur est le facteur de nécrose de tumeur $\alpha$ ou le facteur de nécrose de tumeur $\beta$.

9. Utilisation suivant la revendication 6, dans laquelle la composition est formulée pour une perfusion dans le tissu.

10. Utilisation suivant la revendication 6, dans laquelle la composition comprend également un excipient pharmaceutiquement acceptable.

11. Utilisation suivant la revendication 6, dans laquelle la composition comprend en outre un anti-oxydant ou agent fibrinolytique.

12. Utilisation suivant la revendication 6, dans laquelle le tissu est choisi dans le groupe consistant en le tissu musculaire, le tissu conjonctif, le tissu épithélial, le tissu nerveux, le tissu vasculaire, le tissu osseux, le tissu cérébral et les tissus des organes reproducteurs, des organes respiratoires, des organes digestifs, des organes excréteurs, des organes urinaires, des organes des sens et des organes musculaires squelettiques.

13. Tissu excisé perfusé destiné à une transplantation, comprenant un tissu et une solution de perfusion, ladite solution comprenant un agent protecteur choisi parmi ceux indiqués dans la revendication 1.

14. Tissu suivant la revendication 13, dans lequel le facteur de nécrose de tumeur (TNF) est la TNF-$\alpha$ ou le TNF-$\beta$.

FIG. 1

FIG. 2

FIG. 3A

FIG. 3B

FIG. 4

FIG. 5